# EUROPÄISCHE PATENTSCHRIFT

(11) **EP 0 717 026 B1**
(45) Veröffentlichungstag und Bekanntmachung des Hinweises auf die Patenterteilung: **08.04.1998**
(21) Anmeldenummer: 95119192.3
(22) Anmeldetag: 06.12.1995
(51) Int. Cl.: C07C 45/60, C07C 47/12

(54) **Verfahren zur Herstellung von Glutardialdehyd**
Process for the preparation of glutaraldehyde
Procédé pour la préparation du glutaraldéhyde

(30) Priorität: 15.12.1994 DE 4444709
(43) Veröffentlichungstag der Anmeldung: 19.06.1996
(73) Patentinhaber: BASF AKTIENGESELLSCHAFT, 67056 Ludwigshafen (DE)
(72) Erfinder: Kneuper, Heinz-Josef, Dr., D-68163 Mannheim (DE); Becker, Rainer, Dr., D-67098 Bad Dürkheim (DE); Gehrer, Eugen, Dr., D-67069 Ludwigshafen (DE); Schossig, Juergen, Dr., D-67136 Fussgönheim (DE); Henne, Andreas, Dr., D-67433 Neustadt (DE)

(56) Entgegenhaltungen:
- EP-A- 0 066 224
- EP-A- 0 168 761
- US-A- 2 546 018

## Beschreibung

Die vorliegende Erfindung betrifft ein Verfahren zur Herstellung von Glutardialdehyd durch Umsetzung von Alkoxydihydropyranen mit Wasser in Gegenwart von mikroporösen kristallinen Aluminiumsilikaten mit großen Poren als Katalysator.

Aus der US-A-2,546,018 ist die thermische Hydrolyse von Alkoxydihydropyranen bei Temperaturen von 100 bis 200°C zu Glutardialdehyd bekannt. Zur Erniedrigung der Reaktionstemperatur werden saure Katalysatoren vorgeschlagen.

Homogene Säurekatalyse mit z.B. H₃PO₄ ist aus JP 7226488 bekannt. Die Verwendungen dieser homogenen Säuren als Katalysatoren für die Hydrolyse des Alkoxypyrans zu Glutardialdehyd haben den Nachteil, daß sie nach beendeter Umsetzung im System verbleiben und daher neutralisiert werden müssen. In dieser Form verursachen sie Verfärbungen des Glutardialdehyds bzw. sie katalysieren die Polymerisation des Glutardialdehyds und bewirken damit unerwünschte Trübungen durch Polymerabscheidungen. Die Polymerisationsneigung des Glutardialdehyds ist bekannt und wird im allgemeinen durch Handhabung in wäßrig-verdünnter Lösung weitgehend unterdrückt. Aufgrund dieser Problematik wurden verschiedene Verfahren unter anderem unter Verwendung von sauren Ionentauschern als Hydrolysekatalysatoren beschrieben (EP-A-66 224, US-A-4 244 876 und US-A-4 448 977). Dort wird die Umsetzung von Alkoxydihydropyran mit Wasser oder Alkohol zu Hydroxyalkoxytetrahydropyran oder zu Dialkoxytetrahydropyran als stabile Glutardialdehyd-Vorstufe beschrieben. Die Umsetzung zum gewünschten Glutardialdehyd muß also anschließend noch durchgeführt werden. Neben diesem Nachteil sind hier nicht ausreichende Katalysatoraktivität bzw. -standzeit der Ionentauscher unbefriedigend.

Der vorliegenden Erfindung lag daher die Aufgabe zugrunde, den zuvor genannten Nachteilen abzuhelfen.

Demgemäß wurde ein neues und verbessertes Verfahren zur Herstellung von Glutardialdehyd durch Umsetzung von Alkoxydihydropyranen der allgemeinen Formel I 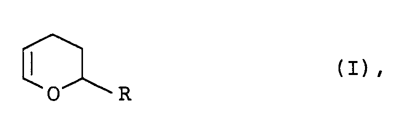 in der R für C₁- bis C₂₀-Alkoxy steht, mit Wasser bei Temperaturen von 0 bis 150°C und Drücken von 0,01 bis 50 bar in Gegenwart eines Katalysators gefunden, welches dadurch gekennzeichnet ist, daß man als Katalysatoren mikroporöse kristalline Aluminiumsilikate mit einem Porendurchmesser von größer als 5,4 Å einsetzt.

Das erfindungsgemäße Verfahren kann wie folgt durchgeführt werden:

Das Alkoxidihydropyran I und Wasser können bei Temperaturen von 0 bis 150°C, bevorzugt 30 bis 100°C, besonders bevorzugt 40 bis 80°C und Drücken von 0,01 bis 50 bar, bevorzugt 0,1 bis 5 bar, besonders bevorzugt bei Atmosphärendruck (Normaldruck) in Gegenwart eines Heterogenkatalysators zum Glutardialdehyd umgesetzt werden.

Als Heterogenkatalysatoren eignen sich mikroporöse kristalline Aluminiumsilikate mit einem Porendurchmesser von größer als 5,4 Å, also von 5,5 bis 20 Å, bevorzugt von 5,8 bis 15 Å, besonders bevorzugt von 6 bis 12 Å, insbesondere Zeolithe vom Beta-Typ (BEA), sowie Pentasile (ZSM-5, ZBM-10) verwendet.

Beta-Zeolithe wurden erstmals in US-A-3 308 069 beschrieben. Sie lassen sich mittels Tetraethylammoniumhydroxid (TEAOH) bei Temperaturen im Bereich von 100 bis 150°C mit der Zusammensetzung TEAOH : SiO₂ : Al₂O₃ : Na₂O : H₂O kristallisieren, wobei das SiO₂/Al₂O₃-Verhältnis im Bereich von 10 zu 200, das Verhältnis Na₂O/TEAOH von 0 bis 1,0, TEAOH/SiO₂ von 0,1 zu 1,0 und H₂O/TEAOH von 20 bis 75 liegen kann. Sie besitzen ein großporiges, dreidimensionales Porensystem mit 12-gliedrigen Ringen mit Durchmessern von 6,5 X 5,6 und 7,5 X 5,7 Å. Der Constraint Index (Methode zur Bestimmung gemäß US-A-4 016 218) liegt zwischen 0,6 und 2,0. Ihre katalytische Wirkung bei der Herstellung von Glutardialdehyd war bislang unbekannt.

Das Molverhältnis von Wasser zum Alkoxidihydropyran I beträgt in der Regel 0,5:1 bis 100:1, bevorzugt 1:1 bis 20:1, besonders bevorzugt 1:1 bis 10:1.

Der Substituent R in den Alkoxydihydropyranverbindungen wird definiert als C₁- bis C₂₀-Alkoxy, bevorzugt C₁- bis C₈-Alkoxy, besonders bevorzugt C₁- bis C₄-Alkoxy wie Methoxy, Ethoxy, n-Propoxy, iso-Propoxy, n-Butoxy, iso-Butoxy, sec.-Butoxy oder tert.-Butoxy, insbesondere Methoxy oder Ethoxy.

Als Alkoxidihydropyrane eignen sich bevorzugt 2-Alkoxy-3,4-dihydropyrane wie 2-Methoxy-3,4-dihydropyran, 2-Ethoxy-3,4-dihydropyran, 2-n-Propoxy-3,4-dihydropyran, 2-iso-Propoxy-3,4-dihydropyran, 2-n-Butoxy-3,4-dihydropyran, 2-iso-Butoxy-3,4-dihydrorohrpyran, 2-sec.-Butoxy-3,4-dihydropyran und 2-tert.-Butoxy-3,4-dihydropyran, besonders bevorzugt 2-Methoxy-3,4-dihydropyran und 2-Ethoxy-3,4-dihydropyran.

Nach der Verseifungsreaktion kann das erhaltene Rohprodukt beispielsweise destillativ vom entstandenen Methanol befreit und so methanolfreier Glutardialdehyd hergestellt werden.

Glutardialdehyd wird z.B. in der Gerberei oder als Mikrobiozid verwendet.

### Beispiele

### Diskontinuierliche Fahrweise

### Beispiel 1

In einem 3-Halsrührkolben werden 114 g Methoxydihydropyran (1 mol) mit 57 g Wasser (3,2 mol) und 5 g Katalysator Beta-Zeolith der Fa. Uetikon (Zusammensetzung: SiO₂ 91 %, Al₂O₃ 7,8 %, Na₂O 0,5 %, K₂O 0,7 %, BET-Oberfläche 700 m²/g, Porengröße in Å 7,6 x 6,7, 5,5 x 5,5, Teilchengröße 0,2 bis 0,5 mm) bei 50°C gerührt. Der Umsatz beträgt nach 90 min etwa 70 %, nach 5 Std. >99 % (gaschromatographische Kontrolle). Nach 5 Std. wird abgekühlt und vom Katalysator abfiltriert, das Rohgemisch enthält 57 % Glutardialdehyd (titrimetrisch bestimmt).

### Beispiel 2

Katalysatorherstellung: 100 g β-Zeolith werden mit 1,5 l einer 10 %-igen Eisennitratlösung 2 h lang bei 80°C ausgetauscht. Anschließend wird mit Wasser gewaschen, abfiltriert und bei 100 °C getrocknet. In einem 3-Halsrührkolben werden 114 g Methoxydihydropyran (1 mol) mit 57 g Wasser (3,2 mol) und 5 g des oben hergestellten Katalysators bei 50°C gerührt. Der Umsatz beträgt nach 90 min etwa 92 %, nach 3 Std. >99 % (gaschromatographische Kontrolle). Nach 3 Std. wird abgekühlt und vom Katalysator abfiltriert, das Rohgemisch enthält 58 % Glutardialdehyd (titrimetrisch bestimmt).

### Kontinuierliche Fahrweise

### Beispiel 3

Katalysatorherstellung: 220 g β-Zeolith aus dem Beispiel 1 wurden mit 5 % Walocel und 230 g Wasser 45 Minuten lang im Kneter verdichtet. Anschließend wurde die Masse mit 70 bar Preßdruck zu 2 mm Strängen verformt. Diese wurden bei 110°C getrocknet und bei 500°C 16 h lang calciniert. 195 g dieser Stränge wurden mit 3 Liter 20 %-iger NH₄Cl-Lösung bei 80°C 2 h lang ausgetauscht und anschließend mit 10 l Wasser gewaschen. Anschließend wurde ein 2. Austausch mit ebenfalls 3 l 20 %-iger NH₄Cl-Lösung bei 80°C vorgenommen und das Produkt Cl-frei gewaschen. Nach dem Trocknen bei 110°C wurde 5 h lang bei 500°C calciniert.

In einem Rohrreaktor, gefüllt mit 0,5 l Katalysatorsträngen wird durch kontinuierliches Zufahren von 35 ml/h Methoxydihydropyran (0,3 mol) und 15 ml/h Wasser (0,83 mol) bei 60°C und Umpumpen von 12 l/h Reaktionslösung Methoxydihydropyran zu Glutardialdehyd hydrolysiert. Der Reaktoraustrag ist einphasig und klar. Der Austrag enthält zwischen 23,2 und 24,1 % Wasser (bestimmt über Karl-Fischer-Tritration), zwischen 54,9 und 56,8 % Glutardialdehyd (titrimetrische Bestimmung über CO-Zahl), zwischen 0,6 und 2,2 % Methoxydihydropyran und zwischen 14 bis 20 % Methanol sowie verschiedene Acetale (gaschromatographische Bestimmung).

## Patentansprüche

1. Verfahren zur Herstellung von Glutardialdehyd durch Umsetzung von Alkoxydihydropyranen der allgemeinen Formel I 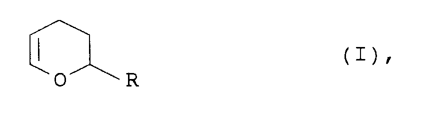 in der R für C₁- bis C₂₀-Alkoxy steht, mit Wasser bei Temperaturen von 0 bis 150°C und Drücken von 0,01 bis 50 bar in Gegenwart eines Katalysators, dadurch gekennzeichnet, daß man als Katalysatoren mikroporöse kristalline Aluminiumsilikate mit einem Porendurchmesser von größer als 5,4 Å einsetzt.

2. Verfahren zur Herstellung von Glutardialdehyd nach Anspruch 1, dadurch gekennzeichnet, daß man als Katalysatoren Zeolithe vom Beta-Typ einsetzt.

3. Verfahren zur Herstellung von Glutardialdehyd durch Umsetzung von Alkoxydihydropyranen der allgemeinen Formel I 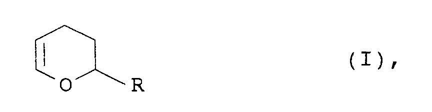 in der R für C₁- bis C₂₀-Alkoxy steht, mit Wasser bei Temperaturen von 0 bis 150°C und Drücken von 0,01 bis 50 bar in Gegenwart eines Katalysators, dadurch gekennzeichnet, daß man als Katalysatoren Pentasil-Zeolithe einsetzt.

4. Verfahren zur Herstellung von Glutardialdehyd nach Anspruch 1, dadurch gekennzeichnet, daß R für C₁- bis C₈-Alkoxy steht.

5. Verfahren zur Herstellung von Glutardialdehyd nach Anspruch 1, dadurch gekennzeichnet, daß R für C₁- bis C₄-Alkoxy steht.

6. Verfahren zur Herstellung von Glutardialdehyd nach Anspruch 1, dadurch gekennzeichnet, daß R für Methoxy oder Ethoxy steht.

7. Verfahren zur Herstellung von Glutardialdehyd nach Anspruch 1, dadurch gekennzeichnet, daß man die Umsetzung bei Temperaturen von 20 bis 120°C durchführt.

8. Verfahren zur Herstellung von Glutardialdehyd nach Anspruch 1, dadurch gekennzeichnet, daß man die Umsetzung bei Drücken von 0,1 bis 10 bar durchführt.

9. Verfahren zur Herstellung von Glutardialdehyd nach Anspruch 1, dadurch gekennzeichnet, daß man die Umsetzung bei Atmosphärendruck durchführt.

## Claims

1. A process for the preparation of glutaraldehyde by reaction of alkoxydihydropyrans of the general formula I 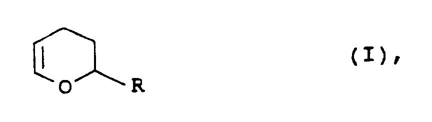 where R is C₁- to C₂₀-alkoxy, with water at from 0 to 150°C and from 0.01 to 50 bar in the presence of a catalyst, wherein the employed catalysts are microporous crystalline aluminum silicates having a pore diameter of greater than 5.4 Å.

2. A process for the preparation of glutaraldehyde as claimed in claim 1, wherein the catalysts employed are zeolites of the beta-type.

3. A process for the preparation of glutaraldehyde by reaction of alkoxydihydropyrans of the general formula I 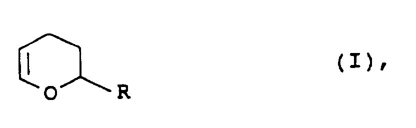 where R is C₁- to C₂₀-alkoxy, with water at from 0 to 150°C and from 0.01 to 50 bar in the presence of a catalyst, wherein the catalysts employed are pentasil zeolites.

4. A process for the preparation of glutaraldehyde as claimed in claim 1, wherein R is C₁- to C₈-alkoxy.

5. A process for the preparation of glutaraldehyde as claimed in claim 1, wherein R is C₁- to C₄-alkoxy.

6. A process for the preparation of glutaraldehyde as claimed in claim 1, wherein R is methoxy or ethoxy.

7. A process for the preparation of glutaraldehyde as claimed in claim 1, wherein the reaction is carried out at from 20 to 120°C.

8. A process for the preparation of glutaraldehyde as claimed in claim 1, wherein the reaction is carried out at from 0.1 to 10 bar.

9. A process for the preparation of glutaraldehyde as claimed in claim 1, wherein the reaction is carried out at atmospheric pressure.

## Revendications

1. Procédé pour la préparation du dialdéhyde glutarique par réaction d'alcoxydihydropyrannes de formule générale I 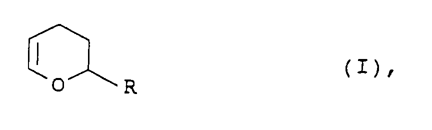 dans laquelle R représente un groupe alcoxy en C1-C20, avec l'eau à des températures de 0 à 150°C et des pressions de 0,01 à 50 bar, en présence d'un catalyseur, caractérisé par le fait que l'on utilise, en tant que catalyseurs, des aluminosilicates cristallins microporeux ayant un diamètre de pore supérieur à 5,4 Å.

2. Procédé pour la préparation du dialdéhyde glutarique selon la revendication 1, caractérisé par le fait que l'on utilise, en tant que catalyseurs, des zéolites du type bêta.

3. Procédé pour la préparation du dialdéhyde glutarique par réaction d'alcoxydihydropyranes de formule générale I 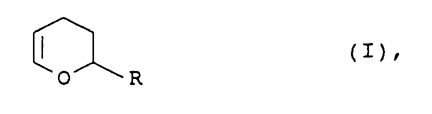 dans laquelle R représente un groupe alcoxy en C1-C20, avec l'eau, à des températures de 0 à 150°C et des pressions de 0,01 à 50 bar en présence d'un catalyseur, caractérisé par le fait que l'on utilise, en tant que catalyseurs, des zéolithes du type pentasil.

4. Procédé pour la préparation du dialdéhyde glutarique selon la revendication 1, caractérisé par le fait que R représente un groupe alcoxy en C1-C8.

5. Procédé pour la préparation du dialdéhyde glutarique selon la revendication 1, caractérisé par le fait que R représente un groupe alcoxy en C1-C4.

6. Procédé pour la préparation du dialdéhyde glutarique selon la revendication 1, caractérisé par le fait que R représente un groupe méthoxy ou éthoxy.

7. Procédé pour la préparation du dialdéhyde glutarique selon la revendication 1, caractérisé par le fait que la réaction est réalisée à des températures de 20 à 120°C.

8. Procédé pour la préparation du dialdéhyde glutarique selon la revendication 1, caractérisé par le fait que la réaction est réalisée sous des pressions de 0,1 à 10 bar.

9. Procédé pour la préparation du dialdéhyde glutarique selon la revendication 1, caractérisé par le fait que la réaction est réalisée à pression atmosphérique.
